(19) Europäisches Patentamt
European Patent Office
Office européen des brevets

(11) Publication number : **0 385 468 B1**

(12) # EUROPEAN PATENT SPECIFICATION

(45) Date of publication of patent specification : **26.08.92 Bulletin 92/35**

(51) Int. Cl.$^5$ : **A61K 31/71,** A61K 47/26, A61K 9/14

(21) Application number : **90104007.1**

(22) Date of filing : **01.03.90**

(54) **A stabilized antitumor composition.**

(30) Priority : **02.03.89 JP 48488/89**

(43) Date of publication of application : **05.09.90 Bulletin 90/36**

(45) Publication of the grant of the patent : **26.08.92 Bulletin 92/35**

(84) Designated Contracting States : **DE FR GB IT**

(56) References cited : **EP-A- 0 275 431**

(73) Proprietor : **MEIJI SEIKA KAISHA LTD.
4-16 Kyobashi 2-chome
Chuo-ku Tokyo 104 (JP)**
Proprietor : **ZAIDAN HOJIN BISEIBUTSU KAGAKU KENKYU KAI
14-23, Kami Ohsaki 3-chome
Shinagawa-ku Tokyo (JP)**

(72) Inventor : **Hosoi, Kaoru, c/o Pharmaceutical Research Center
Meiji Seika Kaisha, Ltd., 760 Morooka-cho
Kohoku-ku, Yokohama-shi, Kanagawa-ken (JP)**
Inventor : **Ishizawa, Takayuki, Pharmaceutical Research Center
Meiji Seika Kaisha, Ltd., 760 Morooka-cho
Kohoku-ku, Yokohama-shi, Kanagawa-ken (JP)**
Inventor : **Okada, Makoto, Pharmaceutical Research Center
Meiji Seika Kaisha, Ltd., 760 Morooka-cho
Kohoku-ku, Yokohama-shi, Kanagawa-ken (JP)**
Inventor : **Yoneta, Toshio, Pharmaceutical Research Center
Meiji Seika Kaisha, Ltd., 760 Morooka-cho
Kohoku-ku, Yokohama-shi, Kanagawa-ken (JP)**
Inventor : **Murata, Shinjiro, Pharmaceutical Research Center
Meiji Seika Kaisha, Ltd., 760 Morooka-cho
Kohoku-ku, Yokohama-shi, Kanagawa-ken (JP)**
Inventor : **Fukatsu, Shunzo, Pharmaceutical Research Center
Meiji Seika Kaisha, Ltd., 760 Morooka-cho
Kohoku-ku, Yokohama-shi, Kanagawa-ken (JP)**
Inventor : **Tsuchiya, Tsutomu
17-201, Eda Higashi 3-chome, Midori-ku
Yokohama-shi, Kanagawa-ken (JP)**

(74) Representative : **Vossius & Partner
Siebertstrasse 4 P.O. Box 86 07 67
W-8000 München 86 (DE)**

**EP 0 385 468 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a stabilized antitumour composition containing an anthracycline compound having an antitumour activity as active ingredient and a stabilizing agent for the active compound and which is useful in the field of medicine This invention also relates to a method for stabilizing an anthracycline compound having the antitumour activity so as to prevent said anthracycline compound from being degraded.

An anthracycline compound having the formula

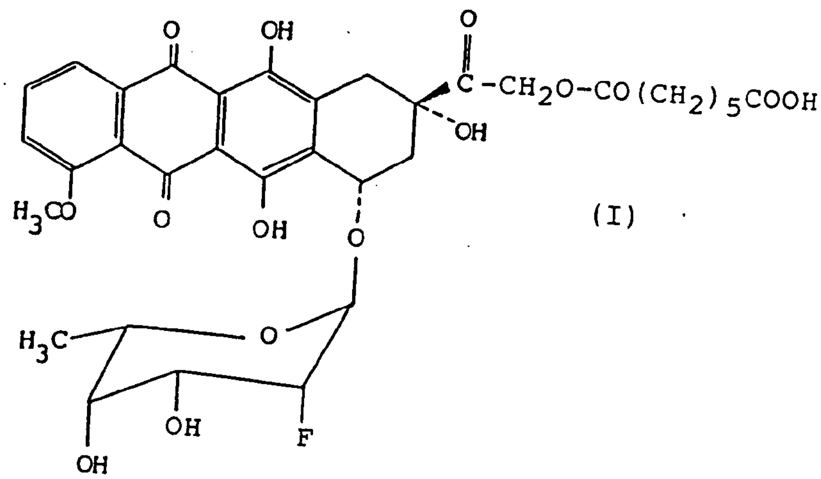

(I)

that is, (8S, 10S)-8-(6-carboxyhexanoyloxyacetyl)-10-[(2,6-dideoxy-2-fluoro-α-L-talopyranosyl)oxy]-7,8,9,10-tetrahydro-6,8,11-trihydroxy-1-methoxy-5,12-naphthacenedione (its abbreviated name: FAD-104 compound) is such an antitumour compound where the fluorine atom possessing a strong electron-withdrawing nature is introduced into the 2-position of the sugar moiety of the antitumor anthracycline antibiotic substance in order to enhance the antitumor effects of said anthracycline substance and where the hydroxyl group at the 14-position is combined with pimelic acid through the ester-linkage in order to improve the solubility of the anthracycline substance in water. This FAD-104 compound is disclosed in JP-A-63-141,992 (Japanese patent application No. Sho-61-288,993) or its corresponding EP-A-275 431 (see Example 5 of said European patent application publication) under a name of 7-O-(2,6-dideoxy-2-fluoro-α-L-talopyranosyl)adriamycinone 14-O-pimelate. The FAD-104 compound and its non-toxic salts may be prepared by the process described in EP-A-275 431.

The FAD-104 compound is expectable as an important antitumour agent in the therapeutic treatment of tumors and cancers in clinics, because the FAD-104 compound can exhibit remarkably enhanced antitumour effects against mouse leukemia L-1210 cells and P-388 cells as well as other tumour cells, as compared to doxorubicin which has widely been utilized in the therapeutic treatment of tumours in clinics, and because the FAD-104 compound can show valuable curative effects against a broader scope of tumors than doxorubicin does. The compound of the above formula (I) in the form of its free carboxylic acid is still somewhat hardly soluble in water, and it is preferred that the compound of formula (I) is administered in the form of its alkali metal salt such as sodium salt and potassium salt or in the form of its ammonium salt or another non-toxic salt of which the solubility in water is increased much more than the compound of the formula (I) in the free carboxylic acid form.

On the other hand, various known antitumour compounds of the anthracycline type which have been utilized in the therapeutic treatment of tumors in the past generally have a poor stability and are readily degradable in their solutions in water, and therefore they have usually been formulated into their medicinal preparations for administration to patients according to such methods of lyophilization or other procedures for formulation by which the degradation of the anthracycline compounds can be prevented or suppressed. It is also always necessary that the medicinal preparations containing such antitumour anthracyclines should be stored in cold places before such medicinal preparations will be administered to patients for clinical treatments of tumours. The compound of formula (I) again does not show a good stability when it is present in its aqueous solution, and accordingly it is normally requested that medicinal preparations containing the compound of formula (I) should be kept in cold places before being provided for the administration to patients in clinics. For this reason, some difficulties can be encountered during commercial production and commercial sales of the medical preparations comprising the compound of formula (I) as active ingredient, so that the use of the compound of formula (I) as a medicinal drug involves some inconvenient problems in practice.

It is the object of this invention to provide a stabilized antitumour composition which comprises the compound of formula (I) or its salt which can show an enhanced stability of the active compound of formula (I) or its salt and is hence storable even at ambient temperatures, whereby practical utility of the active compound of formula (I) for use in the clinical treatment of tumors is improved.

This object has been achieved on the basis of the surprising finding that for the purpose of stabilizing the FAD-104 compound, lactose is selectable as an effective stabilizing agent from amongst the many additives of different natures which have been used and incorporated into the known injectable medicinal preparations as conventionally employed in the therapeutic treatments of patients in clinics, and of which the high safety to humans is well known, and that when the FAD-104 compound of formula (I) is mixed with lactose at the appropriate weight ratios chosen, the compound of formula (I) or a salt thereof can be stabilized remarkedly by providing the presence of lactose in predetermined proportions.

According to a first aspect of this invention, therefore, there is provided a stabilized antitumour composition which comprises a mixture of (8S, 10S)-8-(6-carboxyhexanoyloxyacetyl)-10-[(2,6-dideoxy-2-fluoro-α-L-talopyranosyl)oxy]-7,8,9,10-tetrahydro-6,8,11-trihydroxy-1-methoxy-5,12-naphthacenedione of formula (I) or a non-toxic salt thereof as active ingredient and lactose as the stabilizing agent for the active ingredient compound, with the proportion of lactose being 0.5 to 8 parts by weight of lactose for 1 part by weight of the active ingredient compound of formula (I).

According to a second aspect of this invention, there is provided a method for stabilizing the anthracycline compound of formula (I) from its degradation, which comprises mixing the anthracycline compound of formula (I) or a non-toxic salt thereof with lactose as the stabilizing agent at the weight ratio of the compound of formula (I) or its salt to lactose being in a range of 1 : 0.5 to 1 : 8.

Lactose which is employed according to this invention may be any of hydrated α-lactose, anhydrous α-lactose and anhydrous ß-lactose, or a mixture of two or more of them.

The ratio of the antitumour compound of formula (I) or its non-toxic salt to lactose in the stabilized antitumor composition of this invention is such that 0.5 to 8 parts by weight of lactose is present for 1 part by weight of the antitumour compound of formula (I) or its salt, when lactose as admixed in such proportion can exhibit the effects of stabilizing the anthracycline compound of formula (I) or its salt. It is most preferred that lactose is present in a proportion of 1 to 4 parts by weight per 1 part by weight of the anthracycline compound of formula (I).

The stabilized antitumour composition according to this invention may further contain one or more of any conventional liquid or solid carriers, dissolution-promotors, buffering agents, preservative agents, pH-adjusters, isotonizing agents and/or appropriate excipients, as long as these additional additives do not have any adverse effects against the stabilizing effects of lactose on the FAD-104 compound of formula (I).

It is preferable that, after being mixed with an appropriate amount of lactose, the compound of formula (I) or a salt thereof should be suspended or dissolved in an aqueous buffered solution containing a suitable buffering agent such as potassium dihydrogen phosphate and being adjusted to a pH of 5 to 7, followed by turning the resultant aqueous suspension or solution into a solid form through a known lyophilization method.

In accordance with this invention, the antitumour compound of formula (I) is made to be co-existent with an appropriate proportion of lactose, whereby degradation of the antitumor compound of formula (I) can be substantially prevented from its degradation even when said antitumor compound in the presence of lactose is stored under the conditions of ambient temperatures that are usually prevailing in the courses of the storage and sales of the drug. Hence, even after the stabilized antitumour composition of this invention has been stored at the naturally occurring ambient temperatures which are prevailing during the ordinary courses of the commercial transfers of the drug, the stabilized antitumour composition of this invention is allowable to be provided for actual use in hospitals for the clinical treatment of patients. Thus, the stabilized antitumour composition of this invention can be provided as a medicinal drug which is very much readily handable and utilizable in practice in hospitals for the clinical treatment of tumour-bearing patients. Besides, it is to be noted that when the FAD-104 compound of formula (I) has been degraded, there can be formed a water-insoluble substance which is presumed to mainly comprise adriamycinone that is insoluble in water. Accordingly, when the FAD-104 compound of formula (I) can substantially be prevented from being degraded within the stabilized antitumour composition of this invention owing to the co-existence of lactose,the formation of such water-insoluble substance can be eliminated or minimized, so that the whole of the stabilized antitumour composition of this invention can all be dissolved into a volume of water added in a short time of 5 seconds or less without leaving any residual water-insoluble matters in the water phase. This is of a merit in practice when an injectable aqueous solution is to be prepared in situ by dissolving the stabilized antitumour composition of this invention in a volume of sterile water in hospitals for the clinical treatment of patients.

According to a particular embodiment of this invention, there is provided a method of preparing a stabilized,

lyophilized powdery preparation comprising a sodium or potassium salt of the anthracycline compound of formula (I) as active ingredient and a stabilizingly effective amount of lactose that is completely and readily soluble in water, which method comprises dissolving the compound of formula (I) and lactose into a volume of a buffered aqueous solution containing sodium or potassium hydroxide and potassium phosphate or potassium dihydrogen phosphate at pH 6.4 to 7.4, under sterile conditions, with the weight ratio between the compound of formula (I) and lactose being in a range of 1 : 0.5 to 1 : 8, and then lyophilizing the resulting aqueous solution in a known manner under sterile conditions to produce a stabilized and lyophilized powdery preparation containing the sodium or potassium salt of the compound of formula (I) and lactose as well as the phosphate salt. The lyophilized powdery preparation so produced by the above-mentioned particular embodiment of this invention can advantageously be stored for a long period of time at a temperature of 40°C or less without involving substantial degradation of the antitumour compound of formula (I), and even after a long-time storage, this stabilized and lyophilized powdery preparation is readily soluble in water in a time of 5 seconds or less without leaving any residual water-insoluble matters in the water phase, so that it is readily handable and just ready for formulation of an injectable aqueous solution containing the alkali metal salt of the antitumour compound of formula (I).

This invention is now illustrated with reference to the following Examples.

Example 1

The FAD-104 compound (in the form of free carboxylic acid) (500 mg) was suspended in 20 mℓ of water, and the resulting aqueous suspension was adjusted to pH 7.0 by addition of an aqueous solution of an alkali metal hydroxide indicated in Table 1 below, so that the FAD-104 compound was completely dissolved in the water phase. 2 mℓ-Aliquots of the respective solutions so prepared were placed into glass vials conventionally for use as containers for the injections, so that 50 mg of the FAD-104 compound was charged in each glass vial. The solution in each glass vial was then dehydrated by a conventional method of lyophilization to produce such a lyophilized powdery preparation containing the FAD-104 compound (in the form of the alkali metal salt) which was ready for formulation of injectable aqueous solution by addition of sterile water.

The resulting lyophilized powdery preparations of the FAD-104 compound which were ready for formulation of the aqueous injections were subsequently stored at 70°C for 3 weeks while being kept in the vial containers. After lapse of 3 weeks, the residual content of the FAD-104 compound which was present in the powdery preparation kept in each vial was determined according to a high-performance liquid chromatographic method by assaying the content of the active compound with an ultra-violet absorbency of the tested solution.

The test results obtained are tabulated in Table 1 below.

Table 1

| Alkali metal hydroxide solution added | Content of FAD-104 compound in each vial | |
|---|---|---|
| | Directly after the lyophilization | After storage at 70°C for 3 weeks |
| 5% aqueous solution of sodium hydroxide | 46.3 mg/vial | 33.6 mg/vial |
| 5% aqueous solution of potassium hydroxide | 45.6 mg/vial | 32.7 mg/vial |
| 5% aqueous solution of ammonia | 48.2 mg/vial | 35.5 mg/vial |

The results of Table 1 reveal that the FAD-104 compound can be degraded to a substantial extent when it was processed even in the lyophilization process and was left during the storage at 70°C for 3 weeks (as an accelerated test for estimating the stability of the medicinal compound) if the lactose as the stabilizing agent was absent not in accordance with this invention.

Example 2

The FAD-104 compound (in the form of the free acid) (1 g.) was dissolved in 40 mℓ of 0.15 M buffered aqueous solution of potassium phosphate (at pH 7.4) added, and to the resulting solution was added 2 g of mannitol (as a comparative additive) or 2 g of lactose (as the stabilizing agent according to this invention). The mannitol or lactose dissolved fastly.

The aqueous solution so prepared was placed in 1 mℓ-aliquotes into glass vials conventionally used as the containers for the injectable solutions. The solution in each vial was dehydrated by a conventional method of lyophilization to afford such a lyophilized powdery preparation of the FAD-104 compound which was ready for formulation of injectable solution. The vials containing the powdery preparation of the antitumour compound were stored at 70°C for 3 weeks. With lapse of time during the storage, the content of the FAD-104 compound present in the stored preparation in each vial was determined in the same manner as in Example 1. The content of the FAD-104 compound which was present in the lyophilized preparation in each vial directly after the lyophilization process (namely, at the initial time of the storage period) was then assumed to be 100%. The determination of the content of the FAD-104 compound was made at an interval of one week. The test results obtained are shown in Table 2 below.

Table 2

| Additives | percentages of the residual content of FAD-104 compound in each vial | | | |
|---|---|---|---|---|
| | Directly after the lyophilization | After storage at 70°C for 1 week | After storage at 70°C for 2 weeks | After storage at 70°C for 3 weeks |
| None | 100% | 88% | 88% | 64% |
| Mannitol (as comparative additive) | 100% | 90% | 76% | 72% |
| Lactose (Stabilizing agent according to this invention) | 100% | 99% | 96% | 98% |

Example 3

Respective ingredients indicated in Table 3 below were dissolved in volumes of water in their respective amounts indicated for the formulation ingredients as shown in Table 3, so that there were prepared the starting solutions which were to be lyophilized. These starting solutions were adjusted to pH 6.4 by addition of a 10% aqueous solution of sodium hydroxide and then placed in 2 mℓ-aliquotes into glass vials which were conventionally employed as the containers for injectable solutions. The solution in each vial was lyophilized in a known manner to produce a lyophilized powdery preparation containing the FAD-104 compound. The resulting lyophilized preparation of the FAD-104 compound in each vial was stored at 40°C or at 60°C for 24 weeks. The content of the FAD-104 compound which was present in the lyophilized product directly after the lyophilization process was assumed to be 100 % (as the initial value). With lapse of time during the storage, the residual content of the FAD-104 compound was determined in the same manner as in Example 1. At each time of determination of the content of the FAD-104 compound, the lyophilized preparation under test was removed as the sample from one vial and taken up into 5 mℓ of water. Then, the time that was required for the whole sample of the lyophilized preparation to be completely dissolved in 5 mℓ of water without leaving any residual insoluble matters in the water phase was measured. If the whole sample of the lyophilized preparation of the FAD-104 compound could not be dissolved completely in 5 mℓ of water in a time of 5 seconds or less, it was assumable that the FAD-104 compound could undergo the degradation to a substantial extent, giving a substantial amount of the insoluble substance as the degraded product.

The test results obtained are summarized in Table 4 below.

Table 3

| Formulation Lot No. | Formulation ingredients incoporated per 100 mℓ of the aqueous solution to be lyophilized | | |
|---|---|---|---|
| | FAD-104 compound | Sodium dihydrogen phosphate | Lactose |
| 2-1-3 (Comparative) | 5 g | 3 g | 0 g |
| 2-2-0 (according to this invention) | 5 g | 3 g | 2.5 g |
| 2-2-1 (according to this invention) | 5 g | 3 g | 5 g |
| 2-2-2 ( " " ) | 5 g | 3 g | 10 g |
| 2-2-3 ( " " ) | 5 g | 3 g | 20 g |
| 2-2-4 ( " " ) | 5 g | 3 g | 40 g |
| 2-2-5 (Comparative) | 5 g | 3 g | 60 g |

Table 4

| Formulation Lot No. | Storage temperature (°C) | Percentages of the residual content of FAD-104 compound in each vial in term of the initial value, and the time for the complete dissolution of the stored sample (the values of the required time given in the brackets) | | | |
|---|---|---|---|---|---|
| | | After storage for 2 weeks | After storage for 4 weeks | After storage for 6 weeks | After storage for 24 weeks |
| 2-1-3 (Comparative) | 40° | 99% (<5 seconds) | 99% (<5 seconds) | 97% (<5 seconds) | 91% (>30 seconds) |
| | 60° | 95% (5 seconds) | 89% (>30 seconds) | 87% (>30 seconds) | - |
| 2-2-0 (according to this invention) | 40° | 100% (<5 seconds) | 97% (<5 seconds) | 97% (<5 seconds) | 95% (5 seconds) |
| | 60° | 100% (<5 seconds) | 99% (<5 seconds) | 95% (5 seconds) | - |
| 2-2-1 (according to this invention) | 40° | 100% (<5 seconds) | 100% (<5 seconds) | 97% (<5 seconds) | 99% (<5 seconds) |
| | 60° | 99% (<5 seconds) | 96% (<5 seconds) | 97% (<5 seconds) | - |
| 2-2-2 ( " " ) | 40° | 97% (<5 seconds) | 98% (<5 seconds) | 99% (<5 seconds) | 98% (<5 seconds) |
| | 60° | 98% (<5 seconds) | 97% (<5 seconds) | 97% (<5 seconds) | - |
| 2-2-3 ( " " ) | 40° | 99% (<5 seconds) | 98% (<5 seconds) | 100% (<5 seconds) | 97% (<5 seconds) |
| | 60° | 98% (<5 seconds) | 98% (<5 seconds) | 99% (<5 seconds) | - |
| 2-2-4 ( " " ) | 40° | 97% (<5 seconds) | 99% (<5 seconds) | 98% (5 seconds) | 98% (<5 seconds) |
| | 60° | 98% (<5 seconds) | 100% (<5 seconds) | 98% (5 seconds) | - |
| 2-2-5 (Comparative) | 40° | 99% (<5 seconds) | 96% (5 seconds) | 98% (5 seconds) | 93% (30 seconds) |
| | 60° | 100% (<5 seconds) | 97% (<5 seconds) | 92% (30 seconds) | - |

Note:- The time required for the complete dissolution of the stored preparation which amounts to 30 seconds or more than 30 seconds is generally not acceptable for clinical use in practice.

With regard to the test results of Table 4 above, it can be observed that the comparative sample of the formulation Lot No. 2-1-3 which contained no lactose exhibited a considerable reduction in the residual content of the FAD-104 compound to 91% after the storage at 40°C for 24 weeks, with accompanying the fact that the whole sample could not dissolve completely into water within a time of 30 seconds, indicating that a substantial portion of the FAD-104 compound present had been degraded to give hardly water-soluble matters.

In contrast, the samples of the formulation Lot Nos. 2-2-0 to 2-2-4 which contained lactose as the stabilizing agent according to this invention could exhibit not only the residual content of the FAD-104 compound of at least 95% even after the storage at 40°C for 24 weeks but also their capability that the whole sample after the storage was able to be completely dissolved in water within a time of 5 seconds or less, indicating that lactose can work as the effective stabilizing agent for the FAD-104 compound as long as lactose is mixed with the FAD-104 compound at the weight ratios in a range of 0.5 : 1 to 8 : 1. As to the comparative sample of formulation Lot No. 2-2-5 which contained lactose in the excessive proportion of 12 times as much as the proportion of the FAD-104 compound, it was seen that lactose in this case could exhibit a stabilizing effect on the FAD-104 compound but involve an unfavourable tendency that the time required for the complete dissolution of the sample in water would be prolonged to such an extent that would be not suitable for clinical use in practice.

## Claims

1. A stabilized antitumour composition which comprises a mixture of (8S, 10S)-8-(6-carboxyhexnoyloxyacetyl)-10-[(2,6-dideoxy-2-fluoro-α-L-talopyranosyl)oxy]-7,8,9,10-tetrahydro-6,8,11-trihydroxy-1-methoxy-5,12-naphthacenedione having the formula

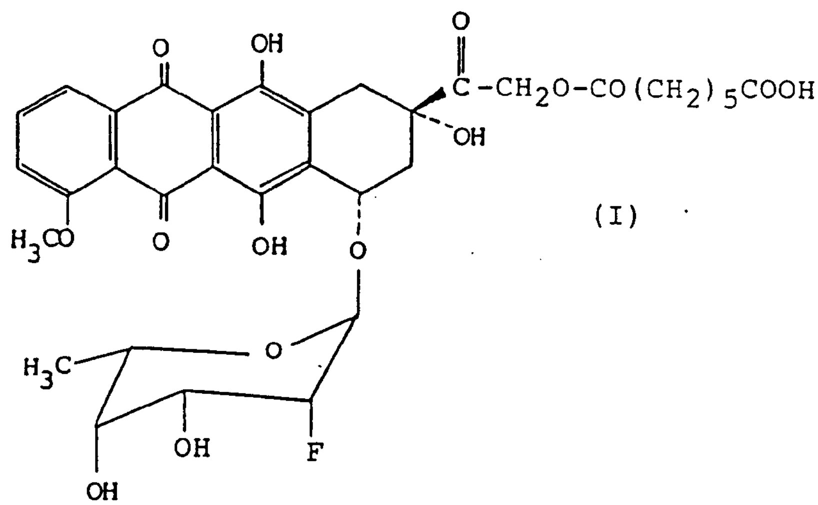

(I)

or a non-toxic salt thereof as active ingredient and lactose as the stabilizing agent for the active ingredient compound, with the proportion of lactose being 0.5 to 8 parts by weight of lactose for 1 part by weight of the active ingredient compound of formula (I).

2. The composition of Claim 1, in which the proportion of lactose is 4 parts by weight for 1 part by weight of the compound of formula (I).

3. A method for stabilizing the anthracycline compound of formula (I) as defined in Claim 1 from its degradation, which comprises mixing the anthracycline compound of formula (I) or a non-toxic salt thereof with lactose as the stabilizing agent at the weight ratio of the compound of formula (I) or a salt thereof to lactose being in a range of 1 : 0.5 to 1 : 8.

4. A method of preparing a stabilized, lyophilized powdery preparation comprising a sodium or potassium salt of the anthracycline compound of formula (I) as defined in Claim 1, as active ingredient and a stabilizingly effective amount of lactose, said preparation being completely and readily soluble in water, which method comprises dissolving the compound of formula (I) and lactose into a volume of a buffered aqueous solution containing sodium or potassium hydroxide and potassium phosphate or potassium dihydrogen

phosphate at pH 6.4 to 7.4, with the weight ratio between the compound of formula (I) and lactose being in a range of 1 : 0.5 to 1 : 8, and then lyophilizing the resulting aqueous solution in a known manner under sterile conditions to produce a stabilized and lyophilized powdery preparation containing the sodium or potassium salt of the compound of formula (I) and lactose as well as the phosphate salt.

## Patentansprüche

1. Stabilisiertes Antitumormittel, umfassend ein Gemisch aus (8S, 10S)-8-(6-Carboxyhexanoyloxyacetyl)-10-[(2,6-didesoxy-2-fluor-α-L-talopyranosyl)oxy]-7,8,9,10-tetrahydro-6,8,11-trihydroxy-1-methoxy-5,12-naphthacendion mit der Formel

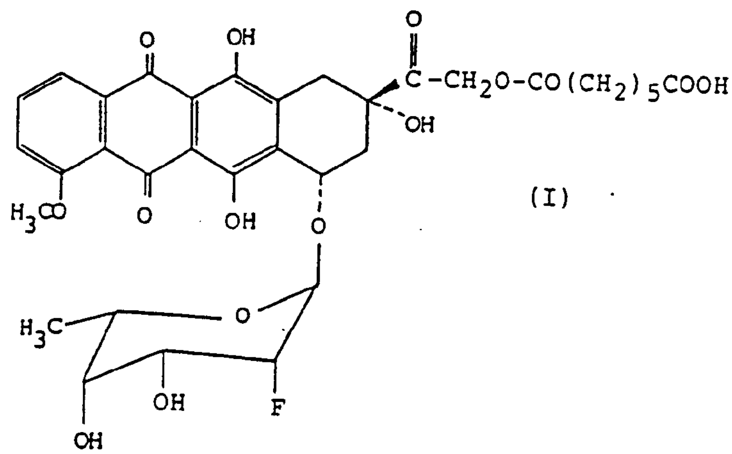

(I)

oder einem nicht-toxischen Salz davon als Wirkstoff und Lactose als Stabilisierungsmittel für die Wirkstoff-Verbindung, wobei der Anteil an Lactose 0,5 bis 8 Gew.- Teile Lactose pro 1 Gew.-Teil der Wirkstoffverbindung der Formel (I) beträgt.

2. Mittel nach Anspruch 1, wobei der Anteil an Lactose 4 Gew. -Teile pro 1 Gew. -Teil der Verbindung der Formel (I) beträgt.

3. Verfahren zur Stabilisierung der Anthracyclin-Verbindung der Formel (I) gemäß Anspruch 1 gegen ihren Abbau, umfassend das Mischen der Anthracyclinverbindung der Formel (I) oder eines nicht-toxischen Salzes davon mit Lactose als Stabilisierungsmittel bei einem Gewichtsverhältnis der Verbindung der Formel (I) oder eines Salzes davon zu Lactose in einem Bereich von 1 : 0,5 bis 1 : 8.

4. Verfahren zur Herstellung eines stabilisierten, gefriergetrockneten pulverförmigen Präparats, umfassend ein Natrium- oder Kaliumsalz der Anthracyclinverbindung der Formel (I) gemäß Anspruch 1 als Wirkstoff und eine stabilisierend wirkende Menge von Lactose, wobei das präparat vollständig und leicht in Wasser löslich ist, umfassend das Lösen der Verbindung der Formel (I) und von Lactose in einem Volumen einer gepufferten wäßrigen Lösung, die Natrium- oder Kaliumhydroxid und Kaliumphosphat oder Kaliumdihydrogenphosphat enthält, bei einem pH-Wert von 6,4 bis 7,4, wobei das Gewichtsverhältnis der Verbindung der Formel (I) und von Lactose in einem Bereich von 1 : 0,5 bis 1 : 8 liegt, und das anschließende Gefriertrocknen der erhaltenen wäßrigen Lösung in an sich bekannter Weise unter sterilen Bedingungen, so daß man ein stabilisiertes und gefriergetrocknetes pulverförmiges Präparat erhält, das das Natrium- oder Kaliumsalz der Verbindung der Formel (I) und Lactose sowie das Phosphatsalz enthält.

## Revendications

1. Composition antitumorale stabilisée, qui comprend un mélange de (8S, 10S)-8-(6-carboxyhexanoyloxyacétyl)-10-[(2,6-didésoxy-2-fluoro-α-L-talopyranosyl)oxy]-7,8,9,10-tétrahydro-6,8,11-trihydroxy-1-métho xy-5, 12-naphtacènedione ayant la formule

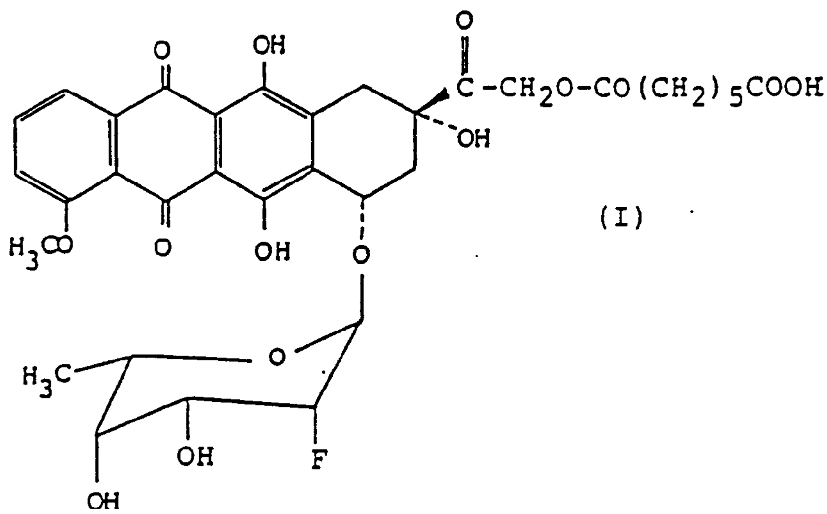

(I)

ou un sel non-toxique de celui-ci comme constituant actif et de lactose comme agent stabilisant pour le constituant actif, la proportion de lactose étant de 0,5 à 8 parties en poids de lactose pour 1 partie en poids du constituant actif de formule (I).

**2.** Composition selon la revendication 1, dans laquelle la proportion de lactose est de 4 parties en poids pour 1 partie en poids du composé de formule (I).

**3.** Procédé pour la stabilisation du composé d'anthracycline de formule (I) tel que défini dans la revendication 1 contre sa dégradation, qui comprend le mélange du composé d'anthracycline de formule (I) ou d'un sel non-toxique de celui-ci avec du lactose en tant qu'agent stabilisant en un rapport en poids entre le composé de formule (I) ou un sel de celui-ci et le lactose compris entre 1 : 0,5 et 1 : 8.

**4.** Procédé pour la production d'une préparation pulvérulente stabilisée, lyophilisée comprenant un sel de sodium ou de potassium du composé d'anthracycline de formule (I) tel que défini dans la revendication 1, en tant que constituant actif, et une quantité de lactose efficace pour la stabilisation, ladite préparation étant totalement et aisément soluble dans l'eau, ledit procédé comprenant la dissolution du composé de formule (I) et du lactose dans un volume d'une solution aqueuse tamponnée contenant de l'hydroxyde de sodium ou de potassium et du phosphate de potassium ou du phosphate monopotassique à un pH de 6,4 à 7,4, le rapport en poids entre le composé de formule (I) et le lactose étant compris entre 1 : 0,5 et 1 : 8, et ensuite la lyophilisation de la solution résultante d'une manière connue dans des conditions stériles pour produire une préparation pulvérulente stabilisée et lyophilisée contenant le sel de sodium ou de potassium du composé de formule (I) et le lactose, ainsi que le sel phosphate.